(19) Europäisches Patentamt
European Patent Office
Office européen des brevets

(11) Numéro de publication: **0 130 898**
**B1**

(12) # FASCICULE DE BREVET EUROPÉEN

(45) Date de publication du fascicule du brevet: **23.09.87**

(51) Int. Cl.⁴: **C 08 F 8/30**, A 61 K 31/795, C 08 B 15/06

(21) Numéro de dépôt: **84401327.6**

(22) Date de dépôt: **25.06.84**

(54) **Produit constitué par un polymère ou un copolymère comportant dans sa chaîne des groupes présentant une affinité vis-à-vis de la thrombine, son procédé de préparation et son utilisation pour la séparation et la purification de la thrombine.**

(30) Priorité: **29.06.83 FR 8310773**

(43) Date de publication de la demande:
**09.01.85 Bulletin 85/2**

(45) Mention de la délivrance du brevet:
**23.09.87 Bulletin 87/39**

(84) Etats contractants désignés:
**AT BE CH DE GB IT LI LU NL SE**

(56) Documents cité:
**EP-A-0 023 854**
**FR-A-2 066 460**

**Le dossier contient des informations techniques présentées postérieurement au dépôt de la demande et ne figurant pas dans le présent fascicule.**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Boisson, Catherine, 16, Place A. Cherioux, F-75015 Paris (FR)**
Inventeur: **Gulino, Danielle, 4, Allée des Jonquilles, F-93360 Neuilly Plaisance (FR)**
Inventeur: **Jozefonvicz, Jacqueline, 64, Deuxième Avenue, F-60260 Lamorlaye (FR)**
Inventeur: **Jozefonvicz, Marcel, 65, Deuxième Avenue, F-60260 Lamorlaye (FR)**

(74) Mandataire: **Mongrédien, André, c/o BREVATOME 25, rue de Ponthieu, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 130 898 B1

LIBER, STOCKHOLM 1987

**Description**

La présente invention a pour objet des produits à base de polymère ou de copolymères substitués par des groupes capables de leur conférer une affinité sélective vis-à-vis de produits intervenant dans le processus de coagulation du plasma sanguin tels que la thrombine et certains facteurs de coagulation comme les facteurs VII et X.

L'invention a également pour objet un procédé de préparation de produits présentant ces caractéristiques et l'utilisation de ces produits pour la séparation et la purification de la thrombine.

De façon plus précise, l'invention concerne des produits à base de polymères ou de copolymères comportant dans leur chaîne des groupes substituables sur lesquels sont fixés de façon statistique des groupes X et Y leur conférant des propriétés particulières.

Des polymères de ce type dans lesquels les groupes X et Y présentent des propriétés anti-coagulantes ont été décrits en particulier dans le brevet français FR-A 2 461 724. Selon ce brevet, on obtient la propriété anticoagulante en fixant sur le polymère un dérivé d'un acide aminé comportant de préférence au moins une fonction carboxylique libre comme l'acide glutamique. Lorsque l'acide aminé comporte plusieurs fonctions amines, il est nécessaire, selon ce brevet, de bloquer toutes les fonctions amines sauf une par un groupe électro-attracteur physiologiquement acceptable tel que benzyloxycarbonyle ou tertiobutyloxycarbonyle, afin d'obtenir une activité anticoagulante satisfaisante supérieure à celle d'un polymère uniquement sulfoné.

Bien qu'ils aient une activité anticoagulante, les produits décrits dans ce brevet français ne présentent pas une affinité sélective pour la thrombine car leur effet sur le processus de coagulation du plasma sanguin est analogue à celui de l'héparine et ils jouent ainsi le rôle de catalyseur. De ce fait, ils ne présentent pas vis-à-vis de la thrombine une affinité et une sélectivité suffisantes pour être utilisés comme adsorbants dans un procédé de séparation et de purification de la thrombine.

On connaît par ailleurs des molécules dérivées de la L-arginine qui présentent une activité inhibitrice vis-à-vis de la thrombine comme cela est décrit par Okamoto et Coll. dans Journal of Medicinal Chemistry, 1980, Vol.23, n°8 p.827 à 836 et p.1293 à 1299. Ces dérivés répondent à la formule:

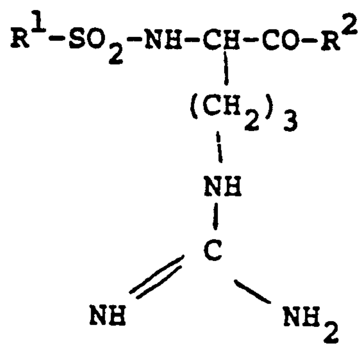

et on étudie l'influence du radical $R^1$ et du radical $R^2$ sur l'activite de ces produits. Cependant, ces auteurs ne donnent aucune indication sur l'affinité et la sélectivité éventuelles de tels produits pour la thrombine.

La présente invention a précisément pour objet des produits à base de polymère ou de copolymère qui présentent une affinité sélective pour la thrombine et peuvent être utilisés de ce fait pour séparer et purifier la thrombine des autres protéines.

Le produit, selon l'invention, est constitué par un polymère ou un copolymère comportant dans sa chaîne des groupes substituables sur lesquels sont fixés de façon statistique des groupes X et Y, dans lequel X représente $-SO_3-R^1$, $R^1$ étant un atome d'hydrogène ou un métal physiologiquement acceptable, et il se caractérise en ce que les groupes Y ont pour formule $-SO_2-R^2$ dans laquelle $R^2$ est un radical dérivé de l'arginine ou d'un dérivé de l'arginine, lié par sa fonction amine -NH- au radical $-SO_2-$.

De préférence, $R^2$ représente le radical de formule:

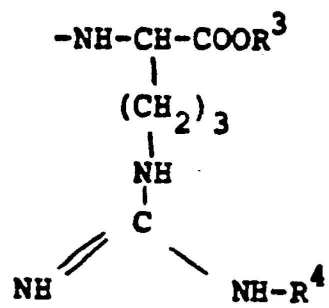

dans laquelle $R^3$ représente l'hydrogène ou un radical alkyle en $C_1$ à $C_4$ et $R^4$ représente l'hydrogène ou le radical $NO_2$.

Selon un mode de réalisation préféré de l'invention, les groupes Y répondent à la formule $-SO_2-R^2$ dans laquelle $R^2$ a la signification donnée ci-dessus avec $R^3$ représentant $CH_3$ et $R^4$ représentant H.

En effet, un tel produit présente une affinité et une sélectivité très importantes pour la thrombine, ce qui le rend particulièrement intéressant. Toutefois, selon l'invention, on peut utiliser également des produits dans lesquels $R^2$ a la signification donnée ci-dessus avec

1. $R^3$ et $R^4$ représentant H,
2. $R^3$ représentant $CH_3$ et $R^4$ représentant $NO_2$, et
3. $R^3$ représentant H et $R^4$ représentant $NO_2$.

Selon l'invention, pour obtenir de bons resultats, il est préférable que la proportion des groupes Y soit de l'ordre de 10 à 20 % par rapport à l'ensemble des groupes X et Y qui sont fixés sur la chaîne du polymère ou du copolymère.

Selon l'invention, les polymères et copolymères susceptibles d'être utilisés sont des matériaux qui présentent le long de leur chaîne des groupes susceptibles de réagir avec l'acide chlorosulfonique.

A titre d'exemple de tels polymères, on peut citer le polystyrène, les esters cellulosiques, les éthers cellulosiques, l'acétate de polyvinyle, le chlorure de polyvinyle, le polyisoprène et le polybutadiène. On peut également utiliser des copolymères obtenus à partir de styrène, d'acétate de vinyle, de chlorure de vinyle, d'isoprène et de butadiène.

De préférence, selon l'invention, le polymère est du polystyrène.

Les produits de l'invention peuvent être, soit des produits solubles dans l'eau et dans les fluides biologiques, soit des produits insolubles dans l'eau et dans les fluides biologiques. Dans ce dernier cas, le polymère ou le copolymère de base portant les groupes X et Y est un polymère ou un copolymère réticulé, ce qui le rend insoluble.

L'invention a également pour objet un procédé de préparation de produits constitués par les polymères ou copolymères substitués décrits ci-dessus.

Ce procédé consiste:

a) à chlorosulfonyler un polymère ou un copolymère en le faisant réagir avec de l'acide chlorosulfonique, et

b) à transformer les groupes $-SO_2Cl$ ainsi fixés sur le polymère ou le copolymère en groupes $-SO_3R^1$ et $-SO_2R^2$ avec $R^2$ représentant un radical dérivé de l'arginine ou d'un dérivé de l'arginine, par réaction du polymère ou du copolymère chlorosulfonylé avec l'arginine ou le dérivé de l'arginine, en milieu basique.

Lorsque le polymère est du polystyrène, la réaction de chlorosulfonylation correspond au schéma réactionnal suivant:

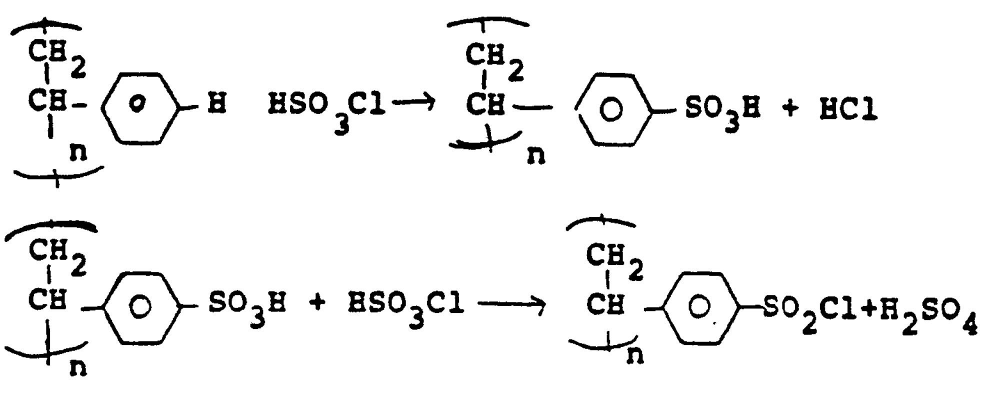

Le polymère de départ peut être un polymère réticulé ou non. Lorsqu'on utilise un polymère non reticulé en vue de préparer des produits solubles, la chlorosulfonylation est réalisée dans un milieu organique

avantageusement constitué par un solvant chloré, par exemple le dichlorométhane, et on précipite ensuite le polymère chlorosulfonylé ainsi obtenu dans du nitrométhane avant de le soumettre à la réaction avec l'arginine ou le dérivé de l'arginine, qui a de préférence lieu dans un milieu contenant un mélange eau-dioxanne pour les acides et un milieu contenant du dichlorométhane pour les dérivés d'esters d'arginine.

En revanche, lorsqu'on désire obtenir des polymères substitués insolubles, on part d'un polymère ou d'un copolymère réticulé et on effectue la réaction dans un mélange contenant un solvant chloré tel que le dichlorométhane, et du nitrométhane, puis on effectue la réaction avec l'acide aminé dans un milieu contenant le mélange eau-dioxanne. Pour la réaction de chlorosulfonylation, on utilise un excès d'acide chlorosulfonique.

La réaction du polymère ou copolymère chlorosulfonylé avec l'arginine ou un dérivé de l'arginine est effectuée dans un milieu contenant un mélange eau-dioxanne. Au cours de la réaction qui s'effectue selon le schéma suivant:

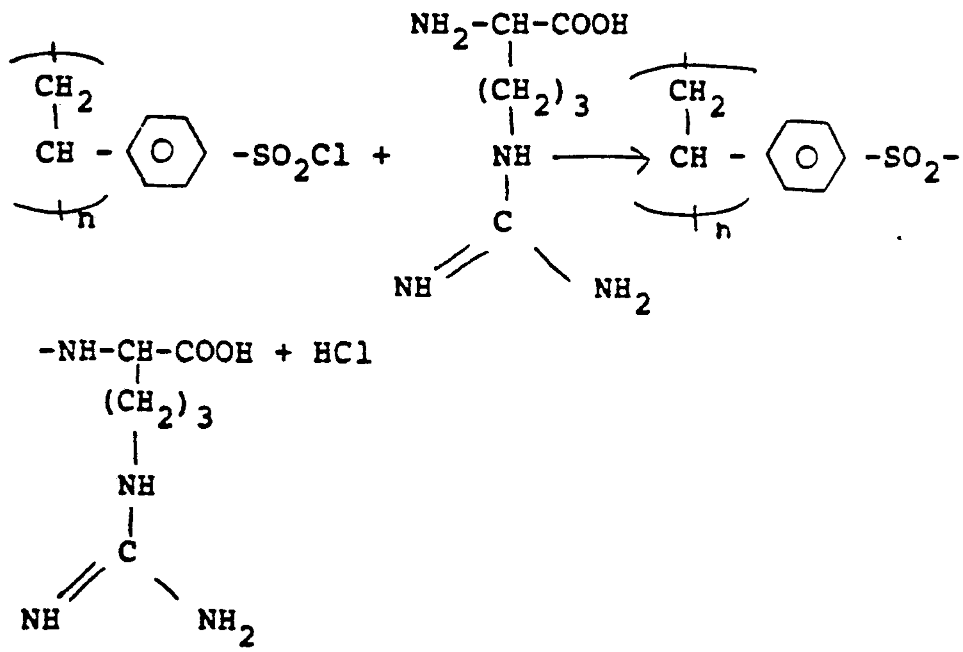

il y a libération d'acide chlorhydrique qui acidifie le milieu, et on maintient le pH du milieu réactionnel à une valeur sensiblement égale à 8 par addition de soude. On peut ainsi suivre l'avancement de la réaction. La réaction est terminée quand le pH demeure constant. Lors de cette réaction, il peut se produire des réactions parasites, en particulier une hydrolyse partielle du polystyrène chlorosulfonylé selon le schéma réactionnel suivant:

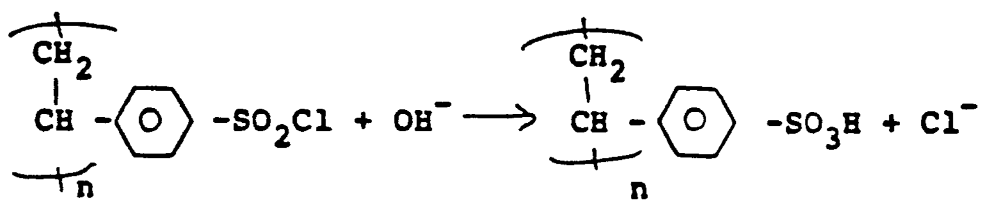

De ce fait, le produit obtenu comportera simultanément des groupes $-SO_2-$ arginine et des groupes $-SO_3H$ ou plus généralement des groupes $-SO_3Na$ puisque la réaction est effectuée en milieu basique, en présence de soude.

Lorsque les groupes $-R^2$ représentent un ester alkylique de l'arginine, on fait réagir le polymère ou le copolymère chlorosulfonylé avec un dichlorhydrate de cet ester méthylique que l'on introduit en solution dans du dichlorométhane. On ajoute alors de la triéthylamine pour débloquer le chlorhydrate, puis on introduit le polymère ou copolymère chlorosulfonylé, de préférence préalablement gonflé par du dichlorométhane, et on ajoute encore petit à petit de la triéthylamine pour neutraliser l'acide chlorhydrique réactionnel.

Après réaction avec l'arginine ou le derive de l'arginine, le polymère ou le copolymère réticulé modifié ainsi obtenu est de préférence soumis à un traitement de purification afin d'éliminer toutes les impuretés qui seraient susceptibles d'interagir avec les facteurs de la coagulation. Par ailleurs, on équilibre de préférence le produit à un pH physiologique.

Ce traitement de purification est généralement réalisé en effectuant plusieurs lavages au moyen d'une

solution de chlorure de sodium, suivis d'un lavage à l'eau distillée, de lavages au citrate de sodium, de lavages au tampon de Michaélis, de rinçages à l'eau distillée et d'un séchage à l'étuve. Après ces opérations, les produits obtenus sont généralement broyés dans un broyeur planétaire afin d'obtenir des particules plus fines car celles-ci se sont révélées plus efficaces pour adsorber la thrombine ou d'autres facteurs de coagulation. Généralement, cette opération est réalisée dans un broyeur planétaire afin d'obtenir des produits ayant une granulométrie moyenne allant de 2 à 100 µm suivant le temps de broyage,

Les produits obtenus par le procédé de l'invention comprennent donc simultanément des groupes $-SO_3R^1$ et des groupes $-SO_2R^2$, et l'on peut faire varier le nombre de groupes $-SO_2-$ dans un large intervalle, par exemple de 1 à 60 % en contrôlant l'avancement de la réaction.

Cependant, pour obtenir des produits présentant une bonne affinité vis-à-vis de la thrombine, il est préférable, dans certains cas, de limiter à des valeurs inférieures à 20 % le nombre de groupes arginine ou dérivés de l'arginine fixés sur le polymère ou le copolymère.

Ainsi, dans le cas du polystyrène comportant des groupes Y dérivés de l'ester méthylique de l'arginine, les meilleurs résultats sont généralement obtenus lorsque le nombre de groupes Y fixés est de 10 à 20 % par rapport à l'ensemble des groupes X et Y.

Les produits de l'invention présentent en particulier une très bonne sélectivité pour la thrombine. De ce fait, lorsque les produits sont insolubles, ils peuvent adsorber sélectivement des quantités importantes de thrombine et la thrombine peut être récupérée ensuite par désorption au moyen de solutions appropriées, par exemple au moyen d'une solution de chlorure de sodium (1,5 M) ou de Polybréne®, c'est-à-dire Le produit ALDRICH: bromure d'hexadiméthrine ou poly-(dibromure de triméthylène-diméthylammonio-hexaméthylène-diméthylammonium). Ceci est très intéressant car on a vérifié que l'activite enzymatique de la thrombine ainsi récupérée était conservée.

De même, les produits de l'invention ont une grande affinité pour les facteurs VII et X intervenant dans le processus de coagulation du sang, et ils présentent aussi la propriété de pouvoir adsorber les anticorps anti VIII C.

De ce fait, les produits de l'invention peuvent trouver de nombreuses applications, ainsi, les produits insolubles dans l'eau et les fluides biologiques peuvent être utilisés pour la réalisation de divers objets tels que des prothèses cardiovasculaires, des cathéters ou des fils de suture en matériau anticoagulant. Les produits solubles dans l'eau et dans les fluides biologiques peuvent être utilisés pour préparer des solutions à action anti-coagulante à usage pharmaceutique.

Toutefois, l'une des applications les plus intéressantes des produits de l'invention, lorsqu'ils sont sous la forme de produits insolubles dans l'eau et dans les fluides biologiques, est la réalisation d'adsorbants sélectits vis-à-vis de la thrombine.

Aussi, l'invention a également pour objet un procédé de séparation et de purification de la thrombine qui consiste à mettre en contact un produit insoluble constitué par un polymère ou un copolymère réticule comportant dans sa chaîne des groupes $-SO_3-R^1$ avec $R^1$ représentant l'hydrogène ou un métal physiologiquement acceptable et des groupes $-SO_2-R^2$ avec $R^2$ représentant un radical provenant de l'arginine ou d'un dérivé de l'arginine lié à $-SO_2-$ par sa fonction amine -NH- avec une solution contenant de la thrombine,

- à séparer de ladite solution le produit ayant adsorbé la thrombine, et
- à récupérer la thrombine par désorption au moyen d'une solution de chlorure de sodium ou de Polybrène®.

De préférence, le produit insoluble utilisé est du polystyrène réticulé comportant des groupes $-SO_3Na$ et

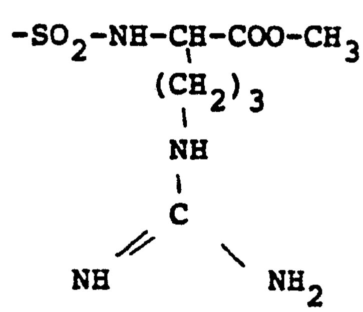

D'autres caractéristiques et avantages de l'invention apparaîtront mieux à la lecture des exemples suivants donnés bien entendu à titre illustratif et non limitatif, en référence au dessin annexé sur lequel:

- les figures 1 à 4 sont des diagrammes illustrant les résultats d'essais de coagulation d'un plasma pauvre en plaquettes ou de fibrinogène, et représentent les variations de la quantité de thrombine inactivée en fonction de la concentration en polystyrène réticulé modifié selon l'invention;
- la figure 5 est un diagramme représentant les variations du taux de thrombine inactivée en fonction de la durée d'incubation;
- la figure 6 représente le taux de thrombine adsorbée en fonction de la quantité de thrombine introduite initialement;
- la figure 7 représente les variations de rapport $\frac{\theta}{1-\theta}$ en fonction de la concentration en thrombine restant dans

le milieu;

- la figure 8 représente les variations du taux de thrombine adsorbée en fonction de la concentration en polystyrène réticulé et modifié selon l'invention; et
- les figures 9 et 10 illustrent les résultats obtenus dans des essais réalisés en présence d'antithrombine III et représentent les variations du pourcentage de thrombine adsorbée en fonction du temps d'incubation.

**Exemple 1**

Cet exemple illustre la préparation de polystyrène réticulé comportant des groupes $SO_3Na$ et des groupes

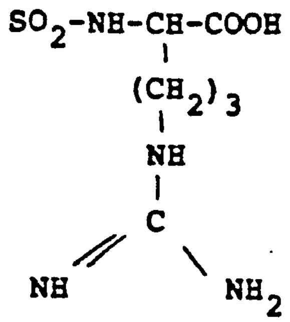

a) <u>synthèse du polystyrène chlorosulfonylé</u>

On met 100 g d'une poudre de polystyrène réticulé ayant une granulométrie de 50 à 130 µm dans 800 ml de NaOH IM, sous agitation douce, à la température ambiante pendant une heure. On filtre la poudre sous vide, puis on la lave à l'eau distillée et on la remet sous agitation douce dans 400 ml de HCl IM, pendant une heure. On filtre la poudre sous vide, on la lave à l'eau distillée, puis au méthanol et on la sèche enfin sous vide à 40°C.

On prend 20 g de la poudre de polystyrène ainsi lavée et on la mélange à 160 ml de $CH_2Cl_2$ dans un ballon sec, puis on la maintient sous agitation douce pendant une heure. On ajoute alors, sous la hotte, 130 ml de nitrométhane et 110 ml de $HSO_3Cl$, puis on met le tout dans un bain d'huile à 40°C pendant 4 heures. On filtre la poudre sous vide et on désactive doucement le filtrat par 50 ml de $CH_2Cl_2$ et d'alcool. On lave alors la poudre au moyen de nitrométhane, puis de $CH_2Cl_2$ et de dioxanne, et on la sèche sous vide à 40°C. On obtient ainsi 20 g de poudre de polystyrène chlorosulfonylé.

b) <u>Fixation du monochlorhydrate d'arginine sur la poudre de polystyrène chlorosulfonylé</u>.

Dans un bécher de 300 ml, on met 20 g de monochlorhydrate d'arginine en présence de 150 ml d'un mélange d'eau et de dioxanne contenant 94 ml d'eau et 56 ml de dioxanne, et on ajoute de la soude NaOH 2M pour solubiliser complètement l'arginine. On ajoute alors 10 g de la poudre de polystyrène chlorosulfonylé obtenue précédemment et on maintient le pH à une valeur de 8-9 en ajoutant de la soude. La réaction est terminée quand le pH ne varie plus. On filtre alors la poudre, on la lave abondamment à l'eau distillée, puis avec une solution de soude NaOH 0,5 M, et on la maintient ensuite dans de l'eau sous agitation douce pendant une nuit. On filtre alors la poudre et on la lave à l'eau distillée, puis on la sèche sous vide à 40°C. On opère de même pour la fixation de la nitroarginine (PNA).

c) <u>Caractérisation</u>

On détermine ensuite les teneurs en azote, en soufre et en chlore du produit obtenu par analyse élémentaire et on détermine le taux de chlore par dosage argentimétrique des ions libérés par hydrolyse. On détermine également la quantité d'arginine fixée en réalisant un dosage acide-base des fonctions carboxyliques, ce qui permet de calculer les pourcentages en sites styrène non modifié (W), sulfonates (X), arginine (Y), et en sous produit

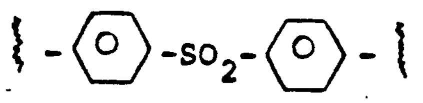 (Z)

des produits obtenus. Les résultats sont donnés dans le tableau 1 joint.

**Exemples 2 à 7**

Ces exemples illustrent la fabrication de polystyrène comportant des groupes $-SO_3Na$ et

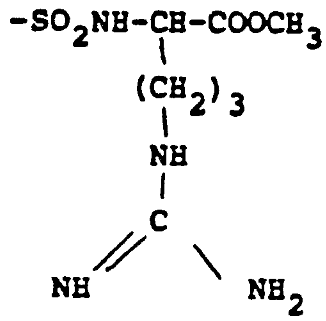

On prépare comme dans l'exemple 1 du polystyrène chlorosulfonylé et on utilise dans chaque exemple 10 g de ce polystyrène chlorosulfonylé.

Dans un ballon bien sec, on dissout sous agitation 16,6 g (64 mmol) de dichlorhydrate de l'ester méthylique d'arginine dans 300 ml de $CH_2Cl_2$. On ajoute ensuite au mélange 29 ml de triéthylamine (200 mmol) pour débloquer le chlorhydrate, et 10 g du polystyrène chlorosulfonylé (42,6 meq de -$SO_2Cl$). On ajoute alors lentement 6 ml de triéthylamine (43 mmol) et on maintient le mélange sous agitation à 20° C pendant 16 heures. On filtre alors le polystyrène sous vide, puis on le lave avec du dioxanne et avec de l'eau et on le sèche sous vide à 40° C.

On soumet les produits obtenus aux mêmes analyses que celles effectuées dans l'exemple 1. Les résultats obtenus figurent sur le tableau 1 joint.

**Exemple 8**

Cet exemple illustre la préparation de polystyrène chlorosulfonylé comportant des groupes -$SO_3Na$ et

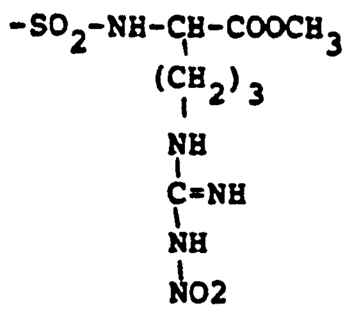

On prépare, comme dans l'exemple 1, du polystyrène chlorosulfonylé ayant une teneur en groupes $SO_2Cl$ de 42,6 meq. On utilise 10 g de ce polystyrène chlorosulfonylé et on le modifie par fixation de l'ester méthylique de nitro-arginine en utilisant le même mode opératiore que celui des exemples 2 à 7, excepté que l'on utilise 17 g (64 mmol) de dichlorhydrate de l'ester méthylique de nitro-arginine au lieu du dichlorhydrate de l'ester méthylique d'arginine. En fin d'opération, on soumet le produit obtenu aux mêmes analyses que celles effectuées dans l'exemple 1 et les résultats obtenus figurent également sur le tableau 1 joint.

**Exemple 9**

Dans cet exemple, on traite les produits obtenus dans les exemples 2 et 3 en vue de les utiliser pour inactiver la thrombine. Tout d'abord, on soumet les produits obtenus à un traitement pour éliminer complètement les impuretés qui seraient susceptibles de réagir avec les protéines du sang. A cet effet, on effectue tout d'abord deux lavages au moyen de NaCl 1,5 M en utilisant 250 ml de NaCl pour 4 g de polystyrène en soumettant le mélange à une agitation douce pendant deux heures. On filtre alors la poudre de polystyrène modifié, puis on fait un léger lavage à l'eau distillée et deux lavages au citrate de trisodium IM, sous agitation douce pendant deux heures. On met ensuite la poudre en suspension pendant deux heures dans du tampon de Michaélis à un pH d'environ 7,32 puis on filtre, on rince abondamment à l'eau distillée et on sèche à l'étuve sous vide. On soumet ensuite la poudre à un broyage pendant trois heures dans un broyeur planétaire et on mesure le diamètre moyen des particules obtenues en utilisant l'observation directe au microscope optique et la

méthode de Coultronix basée sur le mouvement brownien. On détermine enfin le degré de gonflement S de la poudre dans le tampon de Michaelis, qui correspond à:

$$S \text{ (en \%)} = \frac{W-W_0}{W_0} \times 100$$

avec $W_0$ et W représentant respectivement le poids de la poudre sèche et le poids de la poudre hydratée dans un tampon de Michaelis. Les résultats obtenus sont donnés dans le tableau 2 ci-dessous.

**Tableau 2**

| Produit | Granulométrie moyenne (µm) | Degré de gonflement (en %) |
|---|---|---|
| ex.2 | 66 (non broyé) | 2,50 |
| ex..2 | 1,6 (broyé) | |
| ex.3 | 83 (non broyé) | 2,57 |

**Exemple 10**

Dans cet exemple, on teste les propriétés anticoagulantes des produits de l'invention en effectuant les essais suivants:

a) déterminer le temps de coagulation du plasma humain incubé avec une solution contenant des particules de polystyrène réticulé modifié selon l'invention, après avoir déclenché la coagulation par addition de thrombine;

b) déterminer le temps de coagulation du fibrinogène incubé avec la même solution de polymère;

c) déterminer le temps témoin de coagulation du plasma humain en remplaçant la suspension de polystyrène par une solution tampon;

d) déterminer le temps témoin de coagulation du fibrinogène en remplaçant la suspension de polystyrène par une solution tampon; et

e) déterminer le temps de coagulation du fibrinogène ou du plasma en présence d'une solution de polystyrène modifié selon l'invention en déclenchant la coagulation par addition de reptilase.

Dans cet exemple, on utilise le polystyrène réticulé comportant des groupes $-SO_3Na$ et

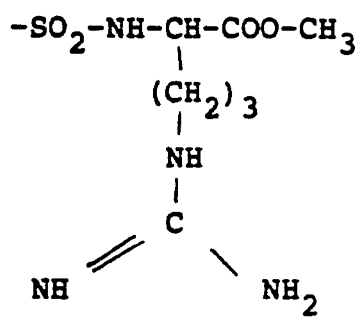

(PAOM) obtenu dans l'exemple 2

Les autres réactifs utilisés sont les suivants:

- Le plasma pauvre en plaquettes (PPP) est préparé à partir de sang humain citraté puis stocké par fractions à une température de -80° C. Les échantillons sont décongelés et stockés à 4° C avant l'essai.

- La thrombine humaine provient du Centre National de Transfusion Sanguine (CNTS) de Paris et elle présente une concentration de 1094 NIH U/mg. On la dissout dans un tampon de Michaélis et on la stocke à -80° C. Elle est maintenue à 4° C avant l'essai.

- Le fibrinogène humain provient également du CNTS de Paris et on le dissout à raison de 6 mg/ml dans une solution de NaCl 0,15 M. Il est maintenu à 37° C pendant l'essai.

- La reptilase fournie par la société Stago est diluée dans de l'eau distillée et maintenue à 37° C avant d'être utilisée.

Les échantillons de polystyrène réticulé comportant des groupes ester méthylique de l'arginine sont mis en suspension dans un tampon de Michaélis et dégazés par ultrasons avant l'essai.

Pour déterminer les différents temps de coagulation, on procède de la manière suivante:

- on incube 0,05 ml d'une suspension de PAOM ou 0,05 ml d'un tampon avec 0,20 ml de plasma humain pauvre en plaquettes (PPP) pendant 10 min à 37° C. On ajoute alors 0,05 ml d'une solution de thrombine (15 NIH U/ml) et on détermine le temps de coagulation à 37° C au moyen d'un coagulomètre KCl.

On effectue des essais dans les mêmes conditions en utilisant une solution de fibrinogène à 6 mg/ml au lieu

du plasma pauvre en plaquettes, et on effectue également des essais de coagulation sur du PPP et sur du fibrinogène dans les mêmes conditions mais en remplaçant la thrombine par de la reptilase.

On effectue encore des essais de coagulation sur du plasma pauvre en plaquettes et sur du fibrinogène en utilisant 0,05 ml de tampon au lieu de 0,05 ml de suspension de polystyrène et en ajoutant 0,05 ml de thrombine à des concentrations variables afin de tracer les courbes d'étalonnage représentant le temps de coagulation du plasma ou du fibrinogène en fonction de la concentration en thrombine libre.

A partir de ces différents résultats, on peut déterminer le taux de thrombine inactivée par le polystyrène modifié par l'ester méthylique de l'arginine (PAOM). On effectue différents essais en faisant varier la concentration en PAOM de la suspension de façon à déterminer le taux de thrombine inactivée en fonction de la concentration en PAOM de la suspension.

Les résultats obtenus sont donnés sur la figure 1 qui représente le taux de thrombine inactivée (en NIH U/ml) en fonction de la concentration en polystyrène de la suspension (en mg/ml). Sur cette figure, la courbe 1 se rapporte aux essais efféctués sur du plasma pauvre en plaquettes et la courbe 2 se rapporte aux essais effectués sur du fibrinogène. On précise que dans ces essais, on a utilisé le PAOM de l'exemple 2 qui a subi un broyage et qui présente une granulométrie moyenne de 1,6 µm.

Sur la figure 2, on a illustré les résultats obtenus dans des essais de coagulation effectués sur du plasma lorsqu'on utilise du PAOM qui n'a pas subi ce broyage et qui présente de ce fait une granulométrie moyenne de 66 ± 3 µm. Cette figure 2 représente également les variations du taux de thrombine inactivée en fonction de la concentration en polymère de la suspension.

En comparant la courbe 1 de la figure 1 avec la courbe de la figure 2, on remarque que l'utilisation d'une poudre de PAOM ayant une dimension de grains moyenne de 1,6 µm permet d'obtenir des taux de thrombine inactivée très supérieurs à ceux que l'on obtient avec le PAOM ayant une granulométrie moyenne de 66 µm. Ainsi, après broyage, le PAOM est capable d'inhiber des quantités importantes de thrombine, ce qui provient sans doute de la proportion plus importante de sites actifs accessibles sur la surface des grains.

Lorsqu'on compare les résultats obtenus sur le plasma et sur le fibrinogène (courbes 1 et 2 de la figure 1), on constate que l'effet anticoagulant du PAOM ne nécessite pas la présence d'inhibiteur de la thrombine tel que l'antithrombine III. Ceci démontre que le PAOM n'a pas un comportement analogue à celui de l'héparine.

Enfin, à la suite de ces essais, on a constaté que les temps de coagulation au moyen de reptilase restaient normaux en présence de la suspension de PAOM. Aussi, on peut en déduire que l'effet anticoagulant du PAOM de l'invention n'est pas dû à une dégradation du fibrinogène.

**Exemple 11**

On effectue les mêmes essais de coagulation en utilisant une suspension de polystyrène modifié par de l'arginine (PA), obtenue dans l'exemple 1 ou de polystyrène modifié par l'ester méthylique de la nitroarginine (PNAOM) obtenue dans l'exemple 8.

Les résultats obtenus sont donnés sur les figures 3 et 4 qui représentent le taux de thrombine inactivée en fonction de la concentration en PA ou PNAOM de la suspension.

La figure 3 se rapporte aux essais effectués sur le plasma et la figure 4 se rapporte aux essais effectués sur du fibrinogène. Les courbes 3 et 3' se rapportent au PNAOM, tandis que les courbes 4 et 4' se rapportent au PA.

**Exemple 12**

Dans cet exemple, on effectue des essais d'adsorption de la thrombine sur le PA de l'exemple 1, le PAOM de l'exemple 2 et le PNAOM de l'exemple 8, en suivant le mode opératoire suivant.

On incube 0,2 ml d'une suspension de PA, PAOM ou PNAOM ou 0,2 ml de tampon de Michaélis avec 0,2 ml de thrombine à des concentrations différentes, pendant des durées différentes, à 37° C. Après décantation, on prélève 0,1 ml du liquide surnageant et on l'ajoute à 0,2 ml de plasma pauvre en plaquettes (PPP). On mesure alors le temps de coagulation et on utilise une courbe d'étalonnage pour déterminer à partir des résultats obtenus dans les essais le taux de thrombine restant dans le liquide surnageant. Le taux de thrombine adsorbé correspond à la différence entre la quantité de thrombine introduite et la quantité de thrombine qui reste dans le liquide surnageant.

Les résultats obtenus avec le PAOM de l'exemple 2 sont donnés sur la figure 5 qui représente le taux de thrombine adsorbée (en U/ml) en fonction de la durée d'incubation (en min). Sur cette figure 5, la courbe 5 se rapporte à des essais effectués avec une suspension de PAOM à une concentration de 4,6 mg/ml et 0,2 ml de thrombine à 50 NIH U/ml; la courbe 6 se rapporte à l'utilisation d'une suspension contenant 15 mg/ml de PAOM et 0,2 ml de thrombine à 25 NIH U/ml, et la courbe 7 se rapporte aux essais effectués avec une suspension contenant 7,5 mg/ml de PAOM et 0,2 ml de thrombine à 10 NIH U/ml.

Les résultats obtenus sur la figure 5 montrent que l'adsorption de la thrombine sur la surface des particules de PAOM augmente avec le temps, mais que la saturation est pratiquement atteinte au bout de 12 min.

Sur la figure 6 on a représenté les isothermes d'adsorption de la thrombine sur les particules de PAOM de l'exemple 2, en réalisant les essais dans les mêmes conditions que précédemment, mais en limitant la durée de mise en contact de la suspension de PAOM avec la thrombine à 12 min. Sur cette figure 6, la courbe 8 illustre les résultats obtenus lorsqu'on utilise une suspension de PAOM contenant 7,5 mg/ml de PAOM et la courbe 9 illustre les résultats obtenus lorsque la teneur en PAOM de la suspension est de 4,6 mg/ml. Ces courbes représentent les variations du taux de thrombine adsorbée en fonction de la concentration initiale en thrombine (en NIH U/ml). Sur cette figure, on remarque que la courbe 9 qui correspond à une teneur en PAOM de 4,6 mg/ml a une pente peu importante au départ et qu'elle atteint un plateau pour une concentration initiale en thrombine d'environ 110 HIH U/ml. Ainsi, on peut en conclure que l'adsorption de thrombine est limitée é 27 NIH U/ml dans les conditions de l'essai et que 1 mg de résine peut adsorber 5,9 NIH D/ml de thrombine. Compte tenu de la dimension des particules de PAOM, on peut supposer que la concentration superficielle en thrombine est de 1,7 ± 0,5 µg/cm². On peut ainsi déterminer le rapport $\frac{\theta}{1-\theta}$ dans lequel θ représente la surface de PAOM couverte par la thrombine. La figure 7 représente les variations de ce rapport en fonction de la concentration en thrombine libre portée en NIH U/ml. A partir de ces résultats, on détermine la constante d'affinité entre le PAOM et la thrombine, et sa valeur qui est indépendante de la granulométrie et de la concentration en PAOM de la suspension est égale à 2,3 ± 0,2 $10^6$ $l.mol^{-1}$.

**Exemple 13**

Dans cet exemple, on effectue également des essais d'adsorption en suivant le même mode opératoire que dans l'exemple 12 mais en utilisant les particules de polystyrène modifiées par l'arginine obtenues dans l'exemple 1 (PA) ayant une dimension de particules de 2 à 70 µm et des particules de polystyrène modifiées par l'ester méthylique de la nitro-arginine (PNAOM) de l'exemple 8 de diamètre 24 µm et des particules de polystyrène modifié par la nitroarginine (PNA) de diamètre: 35 µm, et on détermine le taux de thrombine adsorbée en fonction de la concentration en thrombine initiale ajoutée de la suspension (en NIH-unité/ml).

Les résultats obtenus sont donnés sur la figure 8 qui représente les variations de la concentration de thrombine adsorbée en fonction de la concentration en polymère de la suspension. Sur cette figure, la courbe 10 se rapporte au PA, la courbe 11 se rapporte au PNAOM et la courbe 12 au PNA et la courbe 13 donnée à titre de comparaison représente les résultats obtenus avec des particules de PAOM ayant une granulométrie de 66 µm.

**Exemple 14**

Dans cet exemple, on met en contact en présence ou non d'une suspension de particules de PAOM de l'exemple 2 de la thrombine et de l'antithrombine III qui est un inhibiteur de la thrombine, et on laisse incuber à 37°C pendant des durées variables. On mesure ensuite la quantité de thrombine qui reste dans le liquide surnageant. Les résultats obtenus sont donnés sur les figures 9 et 10 qui représentent le pourcentage (en NIH U/ml) de thrombine desactivée en fonction du temps (en minutes).

Sur la figure 9, la courbe 14 représente les résultats obtenus en présence ou non du PAOM lorsque la quantité d'antithrombine correspond à deux fois la quantité de thrombine, et sur la figure 10, la courbe 15 se rapporte aux résultats obtenus en présence ou non de PAOM lorsque la quantité d'antithrombine représente trois fois la quantité de thrombine.

Ainsi, on constate que la présence de PAOM n'a aucune influence sur l'inhibition de la thrombine par l'antithrombine III, et qu'elle ne joue pas le rôle de catalyseur dans le processus de coagulation.

**Tableau 1**

| Ex. Résine | Poids obtenu (g) | Dosage acide-base (meq/g) | Microanalyse N meq/g | (1) W(%) | (2) X(%) | (3) Y(%) | (4) Z(%) | Taux de gonflement |
|---|---|---|---|---|---|---|---|---|
| 1 PA1 | 14,86 | 1,19 | 1,69 | 16 | 20,9 | 55 | 2 | - |
| 2 PAOM1 | 12,15 | 0,87 | 0,85-0,87 | 16 | 63 | 19 | 2 | 2,50 |
| 3 PAOM2 | 5,66 | 0,96 | 0,96-0,99 | 16 | 60 | 22 | 1 | - |
| 4 PAOM4 | 5,87 | 0,86 | 0,85-0,90 | 16 | 63 | 19 | 1 | - |
| 5 PAOM5 | 11,44 | - | 0,154-0,2 | 16 | 79 | 3,0 | | - |
| 6 PAOM6 | 9,03 | 0,89 | | 16 | 68 | 8,3 | | 2,46 |
| 7 PAOM7 | 13,55 | 0,59-0,52 | 0,35 | 27 | 66 | 6,76 | | 3,00 |
| 8 PNAOM1 | 4,13 | 1,00 | 1,10 | 35 | 40 | 24 | | - |

(1) W = 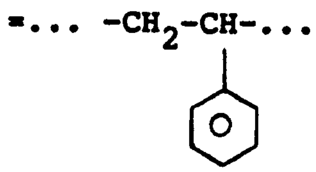

(2) X = 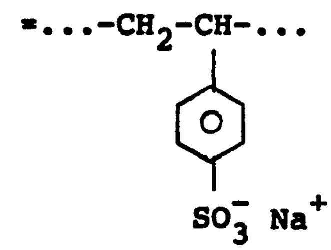

(3) Y = 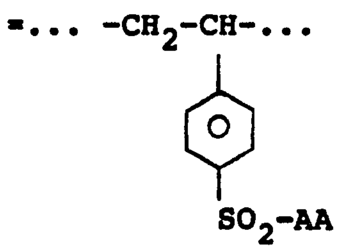

(4) Z = 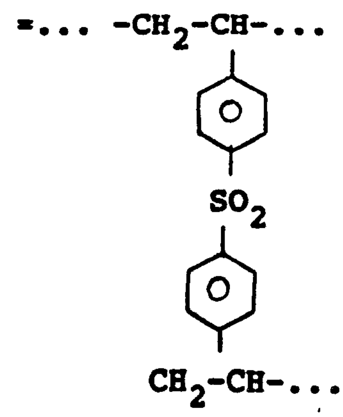

**Revendications** pour les états contractants: BE, CH, DE, GB, IT, LI, LU, NL, SE.

1. Produit constitué par un polymère ou un copolymère comportant dans sa chaîne des groupes substituables sur lesquels sont fixés, de façon statistique, des groupes X et Y, dans lequel X représente $SO_3$-$R^1$ avec $R^1$ étant un atome d'hydrogène ou un métal physiologiquement acceptable, caractérisé en ce que les groupes Y ont pour formule -$SO_2$-$R^2$ dans laquelle $R^2$ représente un radical dérivé de l'arginine ou d'un dérivé de l'arginine, lié par sa fonction amine -NH- au radical -$SO_2$-.

2. Produit selon la revendication 1, caractérisé en ce que $R^2$ représente le radical de formule:

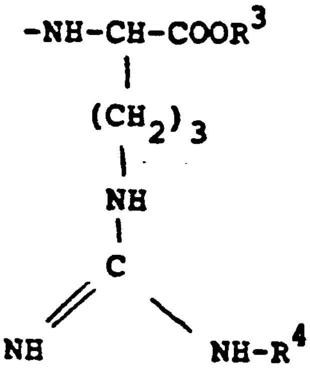

dans laquelle $R^3$ représente l'hydrogène ou un radical alkyle en $C_1$ à $C_4$ et $R^4$ représente l'hydrogène ou le radical $NO_2$.

3. Produit selon la revendication 2, caractérisé en ce que $R^3$ représente $CH_3$ et $R^4$ représente l'hydrogène.

4. Produit selon la revendication 2, caractérisé en ce que $R^3$ et $R^4$ représentent l'hydrogène.

5. Produit selon la revendication 2, caractérisé en ce que $R^3$ représente $CH_3$ et $R^4$ représente $NO_2$.

6. Produit selon la revendication 2, caractérisé en ce que $R^3$ représente l'hydrogène et $R^4$ représente $NO_2$.

7. Produit selon l'une quelconque des revendications 1 à 6, caractérisé en ce que le polymère est choisi parmi le polystyrène, les esters cellulosiques, les éthers cellulosiques, l'acétate de polyvinyle, le chlorure de polyvinyle, le polyisoprène et le polybutadiène.

8. Produit selon la revendication 7, caractérisé en ce que le polymère est du polystyrène.

9. Produit selon l'une quelconque des revendications 1 à 6, caractérisé en ce que le copolymère est un copolymère de styrène, d'acétate de vinyle, de chlorure de vinyle, d'isoprène ou de butadiène.

10. Produit selon l'une quelconque des revendications 1 à 9, caractérisé en ce qu'il est réticule et insoluble dans l'eau.

11. Procédé de préparation d'un produit selon l'une quelconque des revendications 1 à 9, caractérisé en ce qu'il consiste:

a) à chlorosulfonyler un polymère ou un copolymère en le faisant réagir avec de l'acide chlorosulfonique, et

b) à transformer les groupes $-SO_2Cl$ ainsi fixés sur le polymère ou le copolymère en groupes $-SO_3R^1$ et $-SO_2R^2$ avec $R^1$ représentant un métal physiologiquement acceptable, $R^2$ représentant un radical dérivé de l'arginine ou d'un dérivé de celle-ci, par réaction du polymère ou du copolymère chlorsulfonylé avec l'arginine ou un dérivé de l'arginine en milieu basique.

12. Procédé selon la revendication 11, caracterisé en ce que la chlorosulfonylation du polymère ou du copolymère est réalisée dans un milieu comprenant du dichlorométhane.

13. Procédé selon la revendication 11, caractérisé en ce que le polymère ou le copolymère étant réticulé, la chlorosulfonylation est réalisée dans un milieu contenant du dichlorométhane et du nitrométhane.

14. Procédé selon l'une quelconque des revendications 11 à 13, caractérisé en ce que la réaction avec l'arginine ou le dérivé de l'arginine a lieu dans un milieu contenant un mélange d'eau et de dioxanne lorsqu'il s'agit d'arginines acides ou, lorsqu'il s'agit d'esters d'arginine, dans un milieu contenant du dichlorométhane.

15. Procédé de séparation et de purification de la thrombine, caractérisé en ce qu'il consiste à mettre en contact un produit selon l'une quelconque des revendications 1 à 10 avec une solution contenant de la thrombine, à séparer de ladite solution edit produit ayant adsorbé la thrombine et à récupérer la thrombine par désorption au moyen d'une solution de chlorure de sodium ou d'une solution de bromure d'hexaméthrine (Polybrène®).

16. Proxédé selon la revendication 15, caractérisé en ce que ledit produit est du polystyrène reticulé comportant des groupes $-SO_3Na$ et des groupes

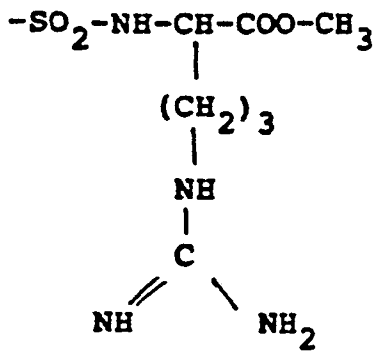

**Revendications** pour l'état contractant AT

1. Procédé de préparation d'un produit constitué par un polymère ou un copolymère sur la chaîne duquel sont fixés de façon statistique des groupes X et Y, caractérisé en ce qu'il consiste:

a) à chlorosulfonyler un polymère ou un copolymère en le faisant réagir avec de l'acide chlorosulfonique, et

b) à transformer les groupes $-SO_2Cl$ ainsi fixés sur le polymère ou le copolymère en groupes X de formule $-SO_3R^1$ et en groupes Y de formule $-SO_2R^2$ avec $R^1$ représentant un atome d'hydrogène ou un métal physiologiquement acceptable et $R^2$ représentant un radical dérivé de l'arginine ou d'un dérivé de celle-ci lié par sa fonction amine -NH- au radical $SO_2$, par réaction du polymère ou du copolymère chlorosulfonylé avec l'arginine ou un dérivé de l'arginine en milieu basique.

2. Procédé selon la revendication 1, caractérisé en ce que la chlorosulfonylation du polymère ou du copolymère est réalisée dans un milieu comprenant du dichlorométhane.

3. Procédé selon la revendication 1, caractérisé en ce que le polymère ou le copolymère étant réticulé, la chlorosulfonylation est realisée dans un milieu contenant du dichlorométhane et du nitrométhane.

4. Procédé selon l'une quelconque des revendications 1 à 3, caracterisé en ce que la réaction avec l'arginine ou le derivé de l'arginine a lieu dans un milieu contenant un mélange d'eau et de dioxanne lorsqu'il s'agit d'arginines acides ou, lorsqu'il s'agit d'esters d'arginine, dans un milieu contenant du dichlorométhane.

5. Procedé selon l'une quelconque des revendications 1 à 4, caractérisé en ce que $R^2$ représente le radical de formule:

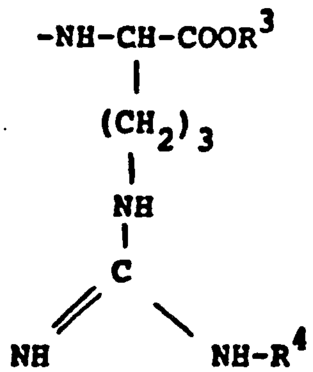

dans laquelle $R^3$ représente l'hydrogène ou un radical alkyle en $C_1$ à $C_4$ et $R^4$ représente l'hydrogène ou le radical $NO_2$.

6. Procédé selon la revendication 5, caractérisé en ce que $R^3$ représene $CH_3$ et $R^4$ représente l'hydrogène.

7. Procedé selon la revendication 5, caractérisé en ce que $R^3$ et $R^4$ représentent l'hydrogène.

8. Procédé selon la revendication 5, caractérisé en ce que $R^3$ représente $CH_3$ et $R^4$ représente $NO_2$.

9. Procédé selon la revendication 5, caractérisé en ce que $R^3$ représente l'hydrogène et $R^4$ représente $NO_2$.

10. Procédé selon l'une quelconque des revendications 1 à 9, caractérisé en ce que le polymère est choisi parmi le polystyrène, les esters cellulosiques, les éthers cellulosiques, l'acétate de polyvinyle, le chlorure de polyvinyle, le polyisoprène et le polybutadiène.

11. Procédé selon la revendication 10, caractérisé en ce que le polymère est du polystyrène.

12. Procédé selon l'une quelconque des revendications 1 à 9, caractérisé en ce que le copolymère est un

copolymère de styrène, d'acétate de vinyle, de chlorure de vinyle, d'isoprène ou de butadiène.

13. Procédé selon l'une quelconque des revendications 1 à 12, caractérisé en ce que le polymère ou le copolymère est réticulé et insoluble dans l'eau.

14. Procédé de séparation et de purification de la thrombine, caractérisé en ce qu'il consiste à mettre en contact le produit obtenu par le procédé selon l'une quelconque des revendications 1 à 13 avec une solution contenant de la thrombine, à séparer de ladite solution ledit produit ayant adsorbé la thrombine et à recupérer la thrombine par desorption au moyen d'une solution de chlorure de sodium ou d'une solution de bromure d'hexaméthrine (Polybrène®).

15. Procédé selon la revendication 14, caractérisé en ce que ledit produit est du polystyrène réticule comportant des groupes $-SO_3Na$ et des groupes

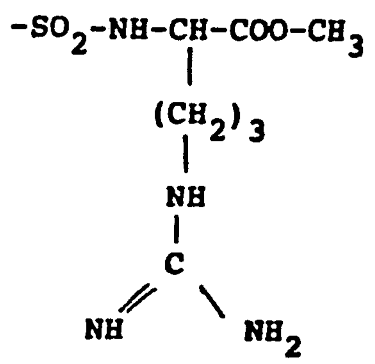

**Patentansprüche** für die Vertragsstaaten BE, CH, DE, GB, IT, LI, LU, NL, SE.

1. Produkt aus einem Polymeren oder einem Copolymeren, das in seiner Kette substituierbare Gruppen aufweist, an, denen in statistischer Weise X- und Y-Gruppen fixiert sind, wobei X $SO_3$-$R^1$ darstellt, worin $R^1$ ein Wasserstoffatom oder ein physiologisch verträgliches Metall bedeutet, dadurch gekennzeichnet, daß die Gruppen Y die Formel $-SO_2$-$R^2$ haben, worin $R^2$ einen von Arginin oder einem Argininderivat abgeleiteten Rest darstellt, der über seine Aminfunktion -NH- an den Rest $-SO_2$- gebunden ist.

2. Produkt nach Anspruch 1, dadurch gekennzeichnet, daß $R^2$ einen Rest der Formel bedeutet

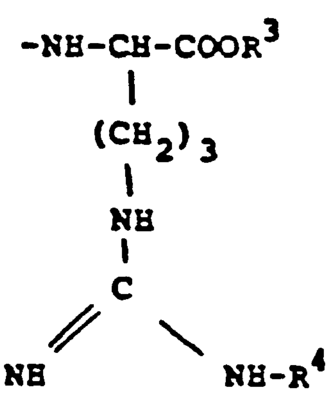

worin $R^3$ ein Wasserstoffatom oder einen $C_1$-$C_4$-Alkylrest und $R^4$ ein Wasserstoffatom oder den $NO_2$-Rest bedeuten.

3. Produkt nach Anspruch 2, dadurch gekennzeichnet, daß $R^3$ $CH_3$ und $R^4$ Wasserstoff bedeuten.

4. Produkt nach Anspruch 2, dadurch gekennzeichnet, daß $R^3$ und $R^4$ Wasserstoff bedeuten.

5. Produkt nach Anspruch 2, dadurch gekennzeichnet, daß $R^3$ $CH_3$ und $R^4NO_2$ bedeuten.

6. Produkt nach Anspruch 2, dadurch gekennzeichnet, daß $R^3$ Wasserstoff und $R^4NO_2$ bedeuten.

7. Produkt nach einem der Ansprüche 1 bis 6, dadurch gekennzeichnet, daß das Polymere ausgewählt wird aus Polystyrol, Celluloseestern, Celluloseäthern, Polyvinylacetat, Polyvinylchlorid, Polyisopren und Polybutadien.

8. Produkt nach Anspruch 7, dadurch gekennzeichnet, daß es sich bei dem Polymeren um Polystyrol handelt.

9. Produkt nach einem der Ansprüche 1 bis 6, dadurch gekennzeichnet, daß es sich bei dem Copolymeren um ein Copolymeres von Styrol, Vinylacetat, Vinylchlorid, Isopren oder Butadien handelt.

10. Produkt nach einem der Ansprüche 1 bis 9, dadurch gekennzeichnet, daß es vernetzt und in Wasser unlöslich ist.

11. Verfahren nach einem der Ansprüche 1 bis 9, dadurch gekennzeichnet, daß man:

(a) ein Polymeres oder ein Copolymeres chlorsulfonyliert, indem man es mit Chlorsulfonsäure reagieren läßt, und

(b) die auf diese Weise an dem Polymeren oder dem Copolymeren fixierten $SO_2Cl$-Gruppen in $-SO_3R^1$- und $-SO_2R^2$-Gruppen umwandelt, worin $R^1$ ein physiologisch verträgliches Metall und $R^2$ einen von Arginin oder einem Argininderivat abgeleiteten Rest bedeuten, durch Umsetzung des chlorsulfonylierten Polymeren oder Copolymeren mit Arginin oder einem Argininderivat in einem basischen Milieu.

12. Verfahren nach Anspruch 11, dadurch gekennzeichnet, daß die Chlorsulfonylierung des Polymeren oder Copolymeren in einem Milieu durchgeführt wird, das Dichlormethan umfaßt.

13. Verfahren nach Anspruch 11, dadurch gekennzeichnet, daß dann, wenn das Polymere oder Copolymere vernetzt ist, die Chlorsulfonylierung in einem Milieu durchgeführt wird, das Dichlormethan und Nitromethan enthält.

14. Verfahren nach einem der Ansprüche 11 bis 13, dadurch gekennzeichnet, daß die Umsetzung mit Arginin oder einem Argininderivat in einem Milieu erfolgt, das eine Mischung aus Wasser und Dioxan enthält, wenn es sich dabei um saure Arginine handelt, oder in einem Dichlormethan enthaltenden Milieu erfolgt, wenn es sich dabei um Argininester handelt.

15. Verfahren zur Abtrennung und Reinigung von Thrombin, dadurch gekennzeichnet, daß man ein Produkt nach einem der Ansprüche 1 bis 10 mit einer Thrombin enthaltenden Lösung in Kontakt bringt, von dieser Lösung das Produkt mit dem daran absorbierten Thrombin abtrennt und das Thrombin durch Desorption mittels einer Lösung von Natriumchlorid oder einer Lösung von Hexametrinbromid (Polybrène®) gewinnt.

16. Verfahren nach Anspruch 15, dadurch gekennzeichnet, daß es sich bei dem Produkt um vernetztes Polystyrol handelt, das $-SO_3Na$-Gruppen und Gruppen der Formel trägt

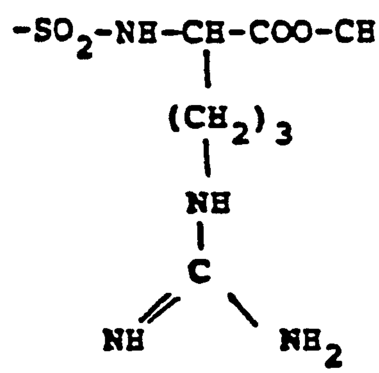

**Patentansprüche** für den Vertragsstaat At

1. Verfahren zur Herstellung eines Produkts aus einem Polymeren oder Copolymeren, an dessen Kette in statistischer Weise X- und Y-Gruppen fixiert sind, dadurch gekennzeichnet, daß man

(a) ein Polymeres oder ein Copolymeres chlorsulfonyliert, indem man es mit Chlorsulfonsäure reagieren läßt, und

(b) die auf diese Weise an dem Polymeren oder dem Copolymeren fixierten $-SO_2Cl$-Gruppen in Gruppen X mit der Formel $-SO_3R^1$ und in Gruppen Y mit der Formel $SO_2R^2$ umwandelt, worin $R^1$ ein Wasserstoffatom oder ein physiologisch verträgliches Metall und $R^2$ einen von Arginin oder einem Argininderivat abgeleiteten Rest, der über seine Aminfunktion -NH- an den Rest $SO_2$ gebunden ist, bedeuten, durch Umsetzung des chlorsulfonylierten Polymeren oder Copolymeren mit Arginin oder einem Argininderivat in basischem Milieu.

2. Verfahren nach Anspruch 1, dadurch gekennzeichnet, daß die Chlorsulfonylierung des Polymeren oder Copolymeren in einem Milieu durchgeführt wird, das Dichlormethan umfaßt.

3. Verfahren nach Anspruch 1, dadurch gekennzeichnet, daß dann, wenn das Polymere oder Copolymere vernetzt ist, die Chlorsulfonylierung in einem Milieu durchgeführt wird, das Dichlormethan und Nitromethan enthält.

4. Verfahren nach einem der Ansprüche 1 bis 3, dadurch gekennzeichnet, daß die Umsetzung mit Arginin oder einem Argininderivat in einem Milieu erfolgt, das ein Gemisch aus Wasser und Dioxan enthält, wenn es sich dabei um saure Arginine handelt, oder in einem Milieu erfolgt, das Dichlormethan enthält, wenn es sich um Argininester handelt.

5. Verfahren nach einem der Ansprüche 1 bis 4, dadurch gekennzeichnet, daß $R^2$ einen Rest der Formel darstellt

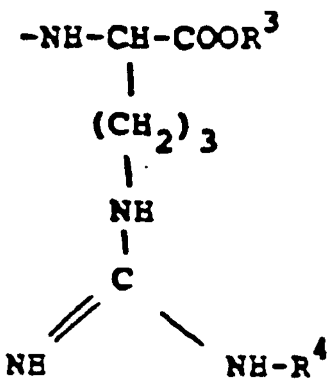

worin $R^3$ Wasserstoff oder einen $C_1$-$C_4$-Alkylrest und $R^4$ Wasserstoff oder den $NO_2$-Rest darstellen.

6. Verfahren nach Anspruch 5, dadurch gekennzeichnet, daß $R^3$ $CH_3$ und $R^4$ Wasserstoff bedeuten.

7. Verfahren nach Anspruch 5, dadurch gekennzeichnet, daß $R^3$ und $R^4$ Wasserstoff bedeuten.

8. Verfahren nach Anspruch 5, dadurch gekennzeichnet, daß $R^3$ $CH_3$ und $R^4$ $NO_2$ bedeuten.

9. Verfahren nach Anspruch 5, dadurch gekennzeichnet, daß $R^3$ Wasserstoff und $R^4$ $NO_2$ bedeuten.

10. Verfahren nach einem der Ansprüche 1 bis 9, dadurch gekennzeichnet, daß das Polymere ausgewählt wird aus Polystyrol, Celluloseestern, Celluloseäthern, Polyvinylacetat, Polyvinylchlorid, Polyisopren und Polybutadien.

11. Verfahren nach Anspruch 10, dadurch gekennzeichnet, daß es sich bei dem Polymeren um Polystyrol handelt.

12. Verfahren nach einem der Ansprüche 1 bis 9, dadurch gekennzeichnet, daß es sich bei dem Copolymeren um ein Copolymeres von Styrol, Vinylacetat, Vinylchlorid, Isopren oder Butadien handelt.

13. Verfahren nach einem der Ansprüche 1 bis 12, dadurch gekennzeichnet, daß das Polymere oder Copolymere vernetzt und in Wasser unlöslich ist.

14. Verfahren zur Abtrennung und Reinigung von Thrombin, dadurch gekennzeichnet, daß man das bei dem Verfahren nach einem der Ansprüche 1 bis 13 erhaltene Produkt mit einer Thrombin enthaltenden Lösung in Kontakt bringt, von dieser Lösung das Produkt, das Thrombin adsorbiert hat, abtrennt und das Thrombin durch Desorption mittels einer Lösung von Natriumchlorid oder einer Lösung von Hexametrinbromid (Polybrène®) gewinnt.

15. Verfahren nach Anspruch 14, dadurch gekennzeichnet, daß es sich bei dem Produkt um vernetztes Polystyrol handelt, das $-SO_3Na$-Gruppen und Gruppen der Formel enthält

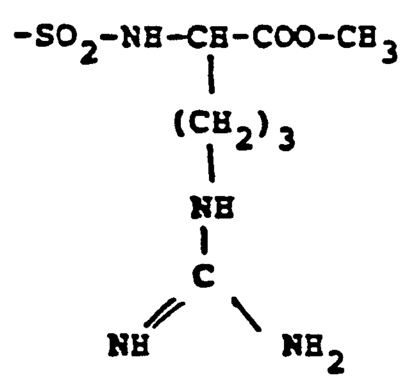

**Claims** for the contracting states BE, CH, DE, GB, IT, LI, LU, NL, SE.

1. Product consisting of a polymer or copolymer containing in its chain groups which can be substituted and to which groups are bound, in statistical fashion, groups X and Y, in which X denotes $SO_3$-$R^1$ with $R^1$ being a hydrogen atom or a physiologically acceptable metal, characterized in that the groups Y have the formula $-SO_2$-$R^2$ in which $R^2$ denotes a radical derived from arginine or from an arginine derivative, linked via its amine group -NH- to the radical $-SO_2$-.

2. Product according to Claim 1, characterized in that $R^2$ denotes the radical of formula:

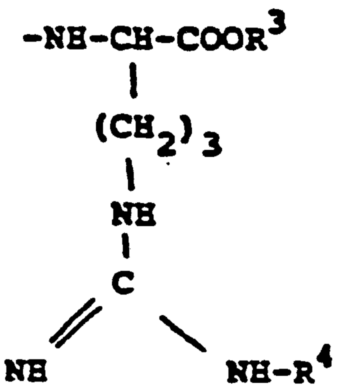

in which $R^3$ denotes hydrogen or a $C_1$ to $C_4$ alkyl radical and $R^4$ denotes hydrogen or an $NO_2$ radical.

3. Product according to Claim 2, characterized in that $R^3$ denotes $CH_3$ and $R^4$ denotes hydrogen.

4. Product according to Claim 2, characterized in that $R^3$ and $R^4$ denote hydrogen.

5. Product according to Claim 2, characterized in that $R^3$ denotes $CH_3$ and $R^4$ denotes $NO_2$.

6. Product according to Claim 2, characterized in that $R^3$ denotes hydrogen and $R^4$ denotes $NO_2$.

7. Product according to any one of Claims 1 to 6, characterized in that the polymer is chosen from polystyrene, cellulose esters, cellulose ethers, polyvinyl acetate, polyvinyl chloride, polyisoprene and polybutadiene.

8. Product according to Claim 7, characterized in that the polymer is polystyrene.

9. Product according to any one of Claims 1 to 6, characterized in that the copolymer is a copolymer of styrene, of vinyl acetate, of vinyl chloride, of isoprene or of butadiene.

10. Product according to any one of Claims 1 to 9, characterized in that it is crosslinked and insoluble in water.

11. Process for preparing a product according to any one of Claims 1 to 9, characterized in that it consists in:

a) chlorosulphonylating a polymer or a copolymer by reacting it with chlorosulphonic acid, and

b) converting the $-SO_2Cl$ groups thereby bound to the polymer or copolymer to $-SO_3R^1$ and $-SO_2R_2$ groups, with $R^1$ denoting a physiologically acceptable metal and $R^2$ denoting a radical derived from arginine or from a derivative of the latter, by reacting the chlorosulphonylated polymer or copolymer with arginine or an arginine derivative in basic medium.

12. Process according to Claim 11, characterized in that the chlorosulphonylation of the polymer or copolymer is carried out in a medium including dichloromethane.

13. Process according to Claim 11, characterized in that where the polymer or copolymer is crosslinked, the chlorosulphonylation is carried out in a medium containing dichloromethane and nitromethane.

14. Process according to any one of Claims 11 to 13, characterized in that the reaction with arginine or the arginine derivative takes place in a medium containing a mixture of water and dioxane when acidic arginines are involved, or in a medium containing dichloromethane when arginine esters are involved.

15. Process for separating and purifying thrombin, characterized in that it consists in bringing a product according to any one of Claims 1 to 10 into contact with a solution containing thrombin, in separating from the said solution the said product which has adsorbed the thrombin, and in recovering the thrombin by desorption by means of a sodium chloride solution or a solution of hexamethrine bromide (Polybrene®).

16. Process according to Claim 15, characterized in that the said product is crosslinked polystyrene containing $-SO_3Na$ groups and groups

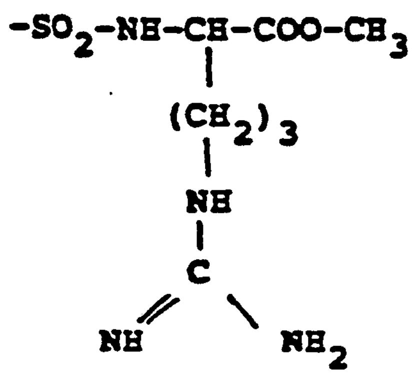

**Claims** for the contracting state: AT

1. Process for preparing a product consisting of a polymer or a copolymer, to the chain of which are bound, in statistical fashion, groups X and Y, characterized in that it consists in:

a) chlorosulphonylating a polymer or a copolymer by reacting it with chlorosulphonic acid, and

b) converting the $-SO_2Cl$ groups thereby bound to the polymer or copolymer to groups X of formula $-SO_3R^1$ and to groups Y of formula $-SO_2R^2$, with $R^1$ denoting a hydrogen atom or a physiologically acceptable metal and $R^2$ denoting a radical derived from arginine or from a derivative of the latter, bound via its amine group -NH- to the radical $SO_2$, by reacting the chlorosulphonylated polymer or copolymer with arginine or an arginine derivative in basic medium.

2. Process according to Claim 1, characterized in that the chlorosulphonylation of the polymer or copolymer is carried out in a medium including dichloromethane.

3. Process according to Claim 1, characterized in that where the polymer or copolymer is crosslinked, the chlorosulphonylation is carried out in a medium containing dichloromethane and nitromethane.

4. Process according to any one of Claims 1 to 3, characterized in that the reaction with arginine or the arginine derivative takes place in a medium containing a mixture of water and dioxane when acidic arginines are involved, or in a medium containing dichloromethane when arginine esters are involved.

5. Process according to any one of Claims 1 to 4, characterized in that $R^2$ denotes the radical of formula:

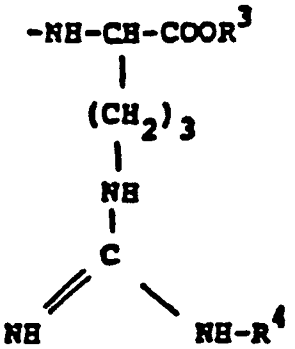

in which $R^3$ denotes hydrogen or a $C_1$ to $C_4$ alkyl radical, and $R^4$ denotes hydrogen or an $NO_2$ radical.

6. Process according to Claim 5, characterized in that $R^3$ denotes $CH_3$ and $R^4$ denotes hydrogen.

7. Process according to Claim 5, characterized in that $R^3$ and $R^4$ denote hydrogen.

8. Process according to Claim 5, characterized in that $R^3$ denotes $CH_3$ and $R^4$ denotes $NO_2$.

9. Process according to Claim 5, characterized in that $R^3$ denotes hydrogen and $R^4$ denotes $NO_2$.

10. Process according to any one of Claims 1 to 9, characterized in that the polymer is chosen from polystyrene, cellulose esters, cellulose ethers, polyvinyl acetate, polyvinyl chloride, polyisoprene and polybutadiene.

11. Process according to Claim 10, characterized in that the polymer is polystyrene.

12. Process according to any one of Claims 1 to 9, characterized in that the copolymer is a copolymer of styrene, of vinyl acetate, of vinyl chloride, of isoprene or of butadiene.

13. Process according to any one of Claims 1 to 12, characterized in that the polymer or copolymer is crosslinked and insoluble in water.

14. Process for separating and purifying thrombin, characterized in that it consists in bringing the product obtained by the process according to any one of Claims 1 to 13 into contact with a solution containing thrombin, in separating from the said solution the said product which has adsorbed the thrombin, and in recovering the thrombin by desorption by means of a sodium chloride solution or a solution of hexamethrine bromide (Polybrene®).

15. Process according to Claim 14, characterized in that the said product is crosslinked polystyrene containing $-SO_3Na$ groups and groups

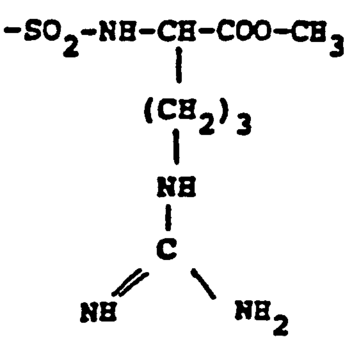

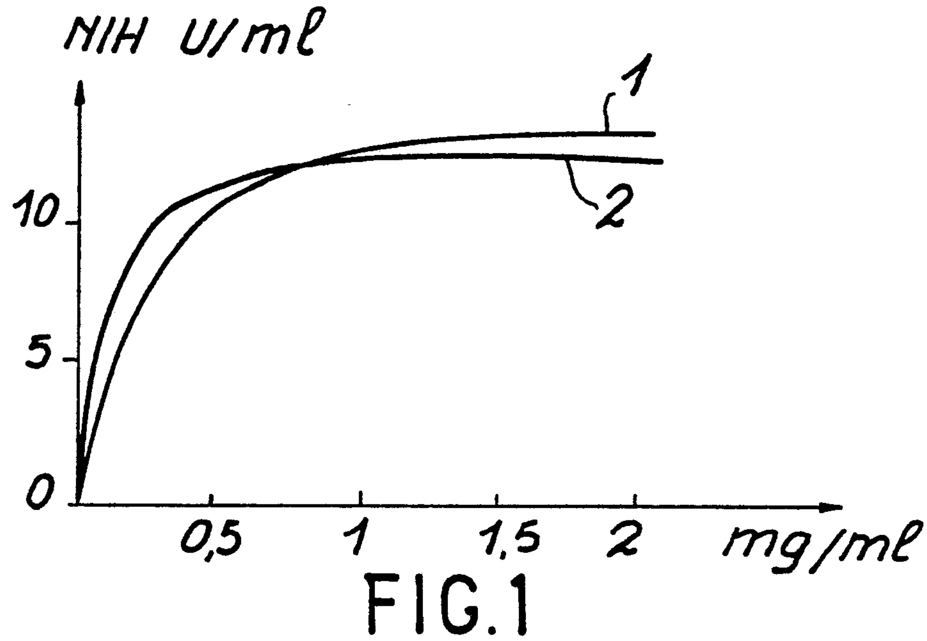


FIG.1

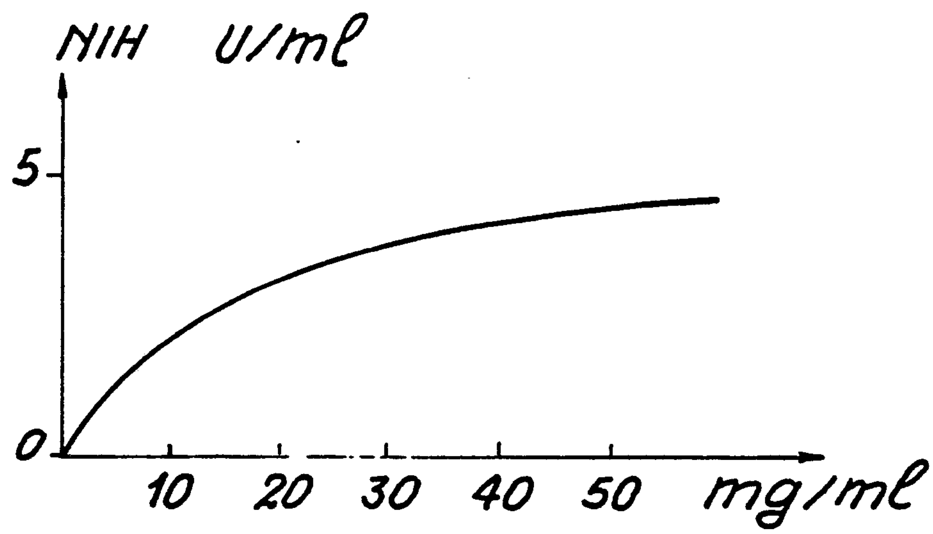


FIG.2

FIG. 3

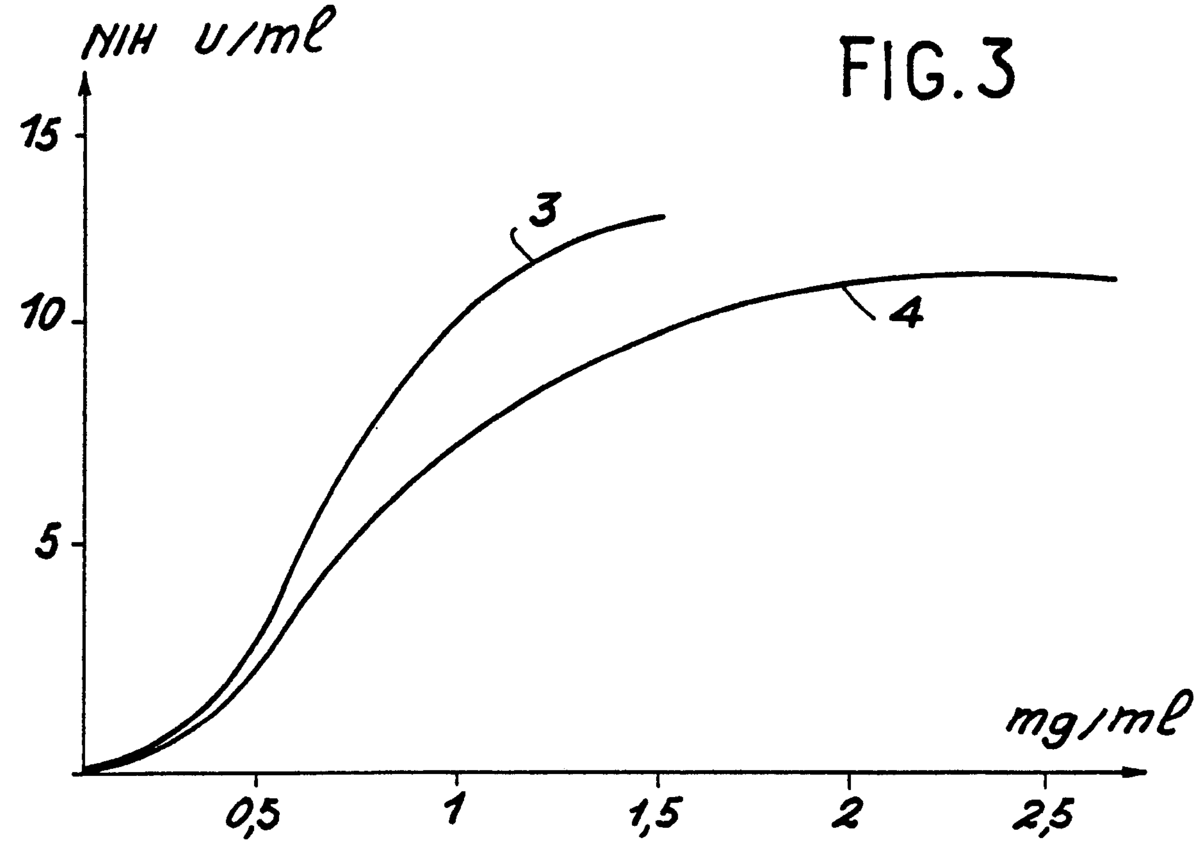

NIH U/ml
15
10
5
3
4
mg/ml
0,5
1
1,5
2
2,5


FIG. 4

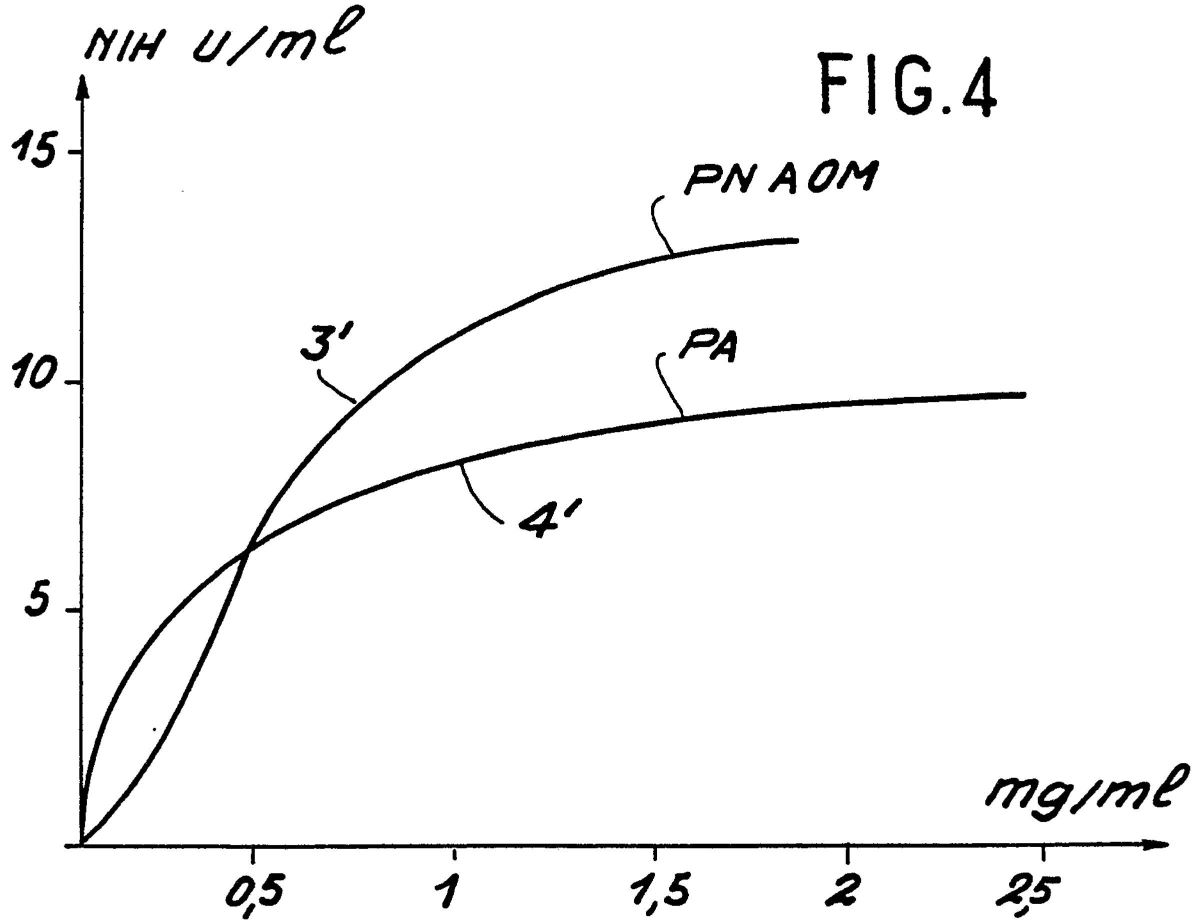

NIH U/ml
15
10
5
PN AOM
3'
PA
4'
mg/ml
0,5
1
1,5
2
2,5

FIG. 5

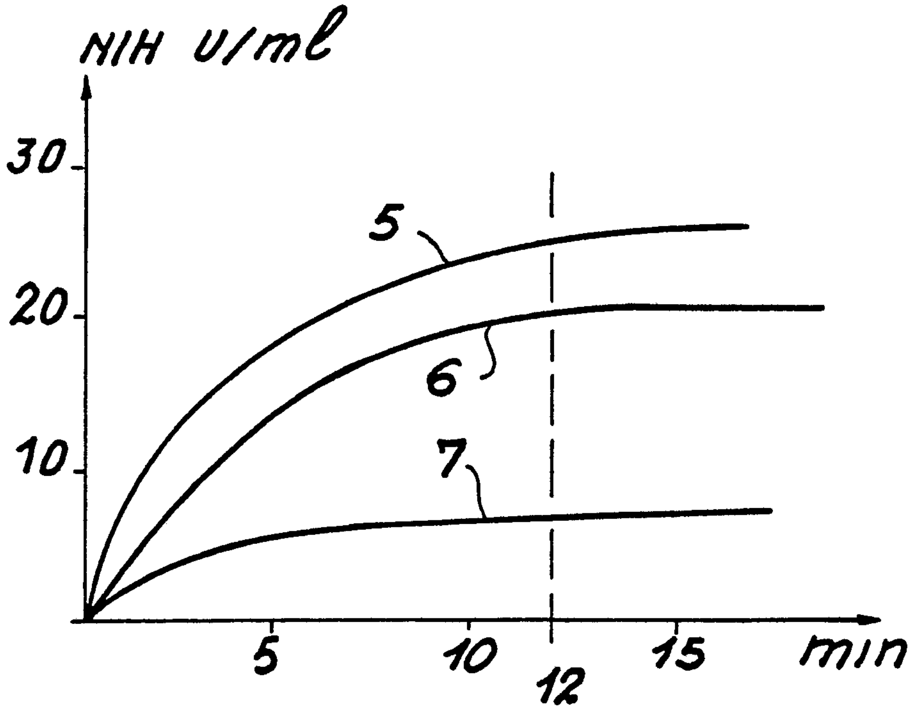


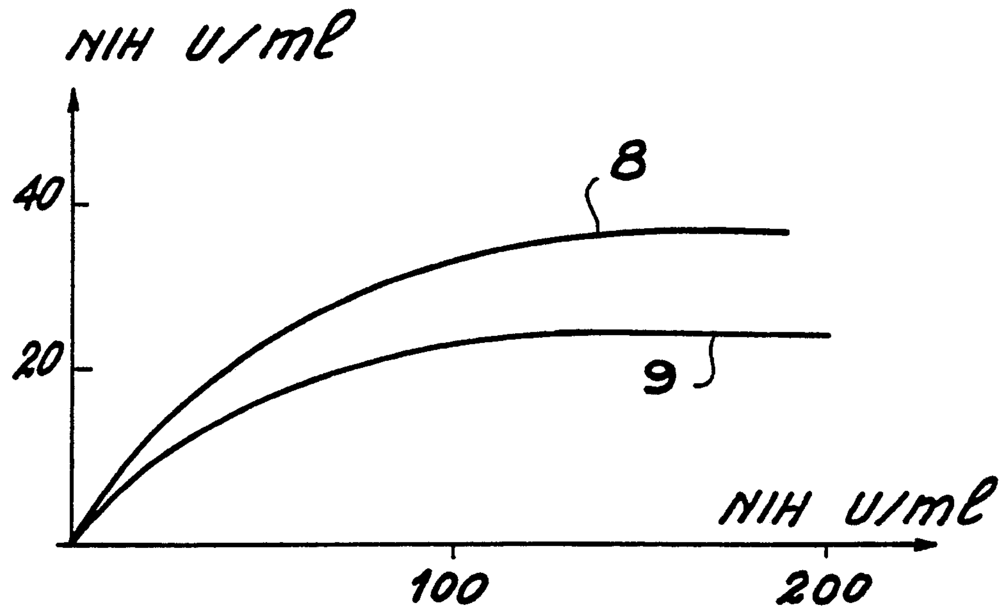


FIG.6

FIG. 7

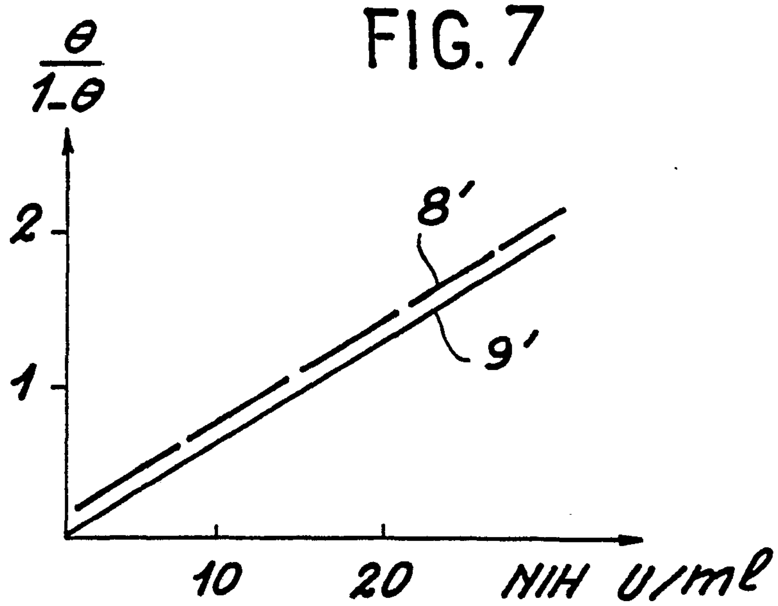


FIG. 8

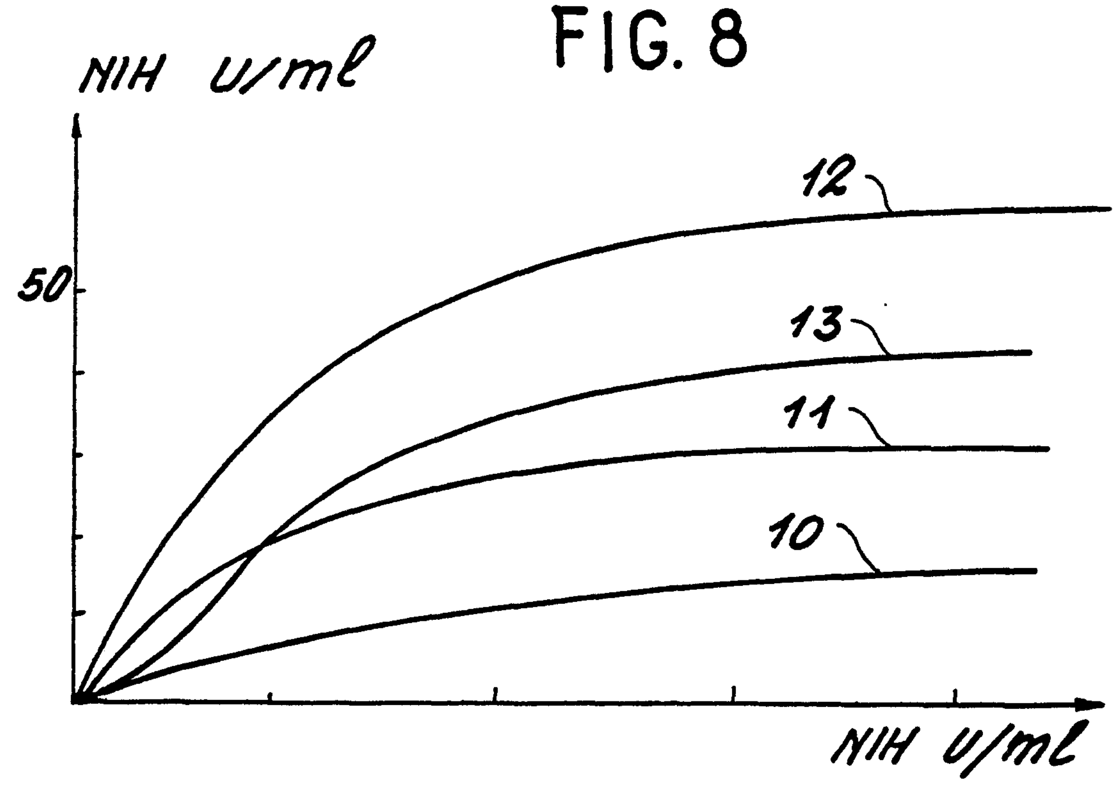

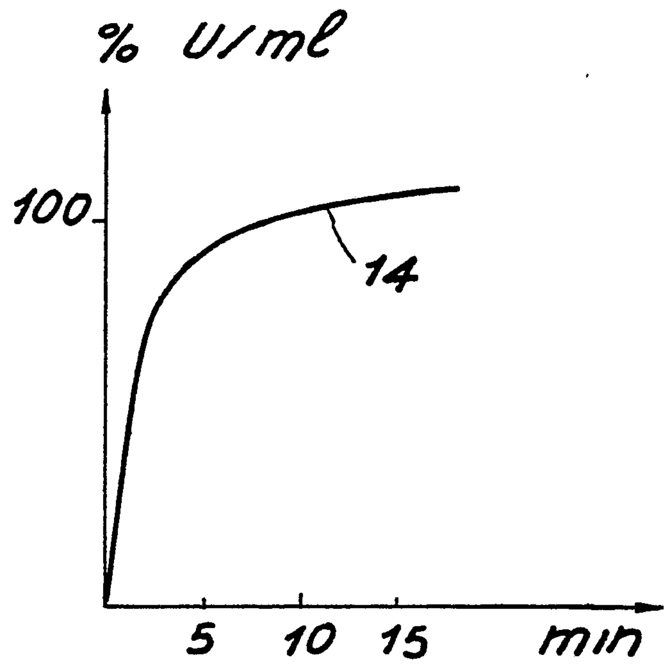


FIG.9

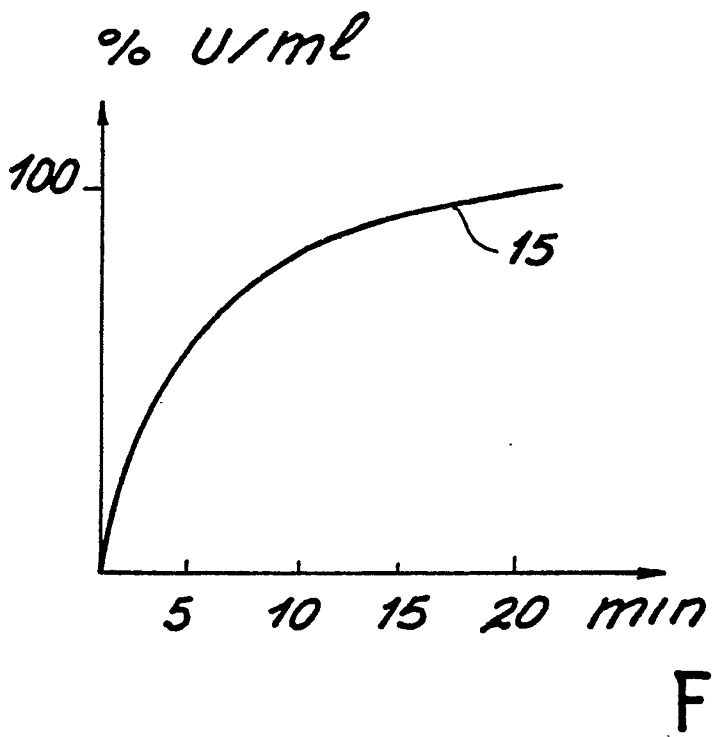


FIG.10